# EUROPEAN PATENT APPLICATION

(11) **EP 3 461 837 A1**
(43) Date of publication of application: **03.04.2019**
(21) Application number: 17193796.4
(22) Date of filing: 28.09.2017
(51) Int. Cl.: C07K 14/47, C12N 15/113

(54) **INHIBITOR INHIBITING THE EXPRESSION OF PPRX1**

(71) Applicant: Secarna Pharmaceuticals GmbH & Co. KG, 35037 Marburg (DE)
(72) Inventor: Jaschinski, Frank, 35037 Marburg (DE); Schirduan, Ksenija, 35037 Marburg (DE); Michel, Sven, 35037 Marburg (DE)
(74) Representative: V.O.

(57) **Abstract**

The present invention refers to an inhibitor consisting of oligonucleotides comprising 10 to 25 nucleotides, wherein at least one of the nucleotides is modified, and the oligonucleotide hybridizes with a nucleic acid sequence of PRRX1 of SEQ ID NO.1 (human) and/or SEQ ID No. 2 (murine), wherein the oligonucleotide inhibits at least 50 % of the PRPPX1 expression. The invention is further directed to a pharmaceutical composition comprising such oligonucleotide.

## Description

The present disclosure refers to an inhibitor of PRRX1 expression consisting of an antisense oligonucleotide hybridizing with a nucleic acid sequence of paired related homeobox 1 (AGOTC, PHOX1, PMX1, PRX-1, PRX1, PRRX1; P54821) and to a pharmaceutical composition comprising such antisense oligonucleotide and a pharmaceutically acceptable carrier, excipient and/or dilutant. Further, the invention refers to the use of the inhibitor or the pharmaceutical composition comprising the inhibitor in a method of preventing and/or treating an inflammatory and/or fibrotic disease.

### Technical background

PRRX1 is part of the paired family of homeobox proteins. PRRX1 functions as a transcription co-activator, enhancing the DNA-binding activity of serum response factor, a protein required for the induction of genes by growth and differentiation factors. PRRX1 also regulates muscle creatine kinase, and therefore has a part in the establishment of diverse mesodermal muscle types. The protein binds to an A/T-rich element in the muscle creatine enhancer.

Alternative splicing of the PRRX1 results in two isoforms-PRRX1a and PRRX1b-that differ in abundance and expression patterns. PRRX1a, the canonical PRRX1 transcript, is translated into a 245-amino-acid protein, whereas PRRXlb encodes for a 217-amino-acid product that is identical from the N terminus to amino acid 199. This overlap includes the homeobox domain (94-153 amino acids). The DNA-binding homeodomain shows similarities to that of other paired families of transcription factors, most notably prd (paired) and gsb (gooseberry); however, PRRX1 lacks the paired domain. At their C termini, PRRX1a and PRRXlb differ from each other. PRRX1a harbors a so-called OAR (otp, aristaless, and rax) domain, named after three proteins that share this 15-amino-acid region. The OAR domain appears to be involved in modifying transactivation ability (Simeone et al. 1994; Norris and Kern 2001). The C-terminal end of PRRXlb lacks the OAR domain and does not contain any other known protein domains and therefore is designated as having an alternative C terminus. Interestingly, the amino acid sequence is 100% conserved in their human orthologs, underscoring their potential biological importance. PRRX1 has an important role during embryonic development, as *Prrx1*^{*-*/*-*} mice die soon after birth. The phenotype of *Prrx1*^{*-*/*-*} mice consists of craniofacial defects, limb shortening, and incomplete penetrant spina bifida (Martin and Olson 2000). PRRX1 may be a regulator of sonic hedgehog and controls cell proliferation during mandibular arch morphogenesis (ten Berge et al. 2001). Functionally, it is known that PRRX1 expression induced by FAK promotes tenascin C-dependent fibroblast migration (McKean et al. 2003).

The PRRX1 gene encodes a transcription factor which is involved in different fibrosis relevant mechanism. An important role of PRRX1 is the transcriptional coordination of differentiation of fibroblasts (Tomaru et al., Nucleic Acids Res. 2014). Due to the inhibition of the expression of PRRX1 on mRNA level via oligonucleotides the development and function of profibrotic cells can be inhibited. This increases for example therapeutic success in the treatment of different chronic fibrotic and chronic inflammatory diseases significantly. Among the diseases associated with PRRX1 are for example idiopathic pulmonary fibrosis, liver fibrosis, kidney fibrosis, agnathia-otocephaly complex, and pleomorphic liposarcoma. The chronic imbalance of fibroblast activity is known as fibrosis and can occur as a central or a concomitant phenotype in multiple diseases. PRRX1 plays a central role in fibroblasts and is therefore a critical player in the progression of diverse fibrotic pathologies.

The intention of the present invention is to provide a reliable specific inhibitor of PRRX1. PRRX1 for example as transcription factor coordinates different processes in fibroblast differentiation:

So far, no antisense oligonucleotide exists which is highly efficient in reduction and inhibition, respectively, of PRRX1 expression. Studies with siRNA - for proof of concept, not for therapeutic investigations - to inhibit PRRX1 expression showed that in *vivo* inhibition is only possible if siRNA is packed in suitable packaging material. Even if siRNA is packed the efficiency on the inhibition of mRNA expression can often not be improved.

An oligonucleotide of the present invention is very successful in the inhibition of the expression of PRRX1. The mode of action of an oligonucleotide differs from the mode of action of an antibody or small molecule, and oligonucleotides are highly advantageous regarding for example
(i) the penetration of tissues such as poorly vascularized tissues,
(ii) the blocking of multiple functions and activities, respectively, of a target,
(iii) the combination of oligonucleotides with each other or an antibody or a small molecule, and
(iv) the inhibition of intracellular effects which are not accessible for an antibody or inhibitable via a small molecule.

### Summary

The present invention refers to an inhibitor of the expression of PRRX1 consisting of an antisense oligonucleotide comprising about 10 to 25 nucleotides, wherein at least one of the nucleotides is modified. The oligonucleotide hybridizes for example with a nucleic acid sequence of PRRX1 protein of SEQ ID NO.1 (human). The modified nucleotide is for example selected from the group consisting of a bridged nucleic acid (e.g., LNA, cET, ENA, 2'Fluoro modified nucleotide, 2'O-Methyl modified nucleotide, a 2'O-Methoxy modified nucleotide, a FANA or a combination thereof). In some embodiments, the oligonucleotide inhibits at least 50 % of the PRRX1 expression and in some embodiments the oligonucleotide inhibits the expression of PRRX1 at a nano- and/or micromolar concentration.

The present invention is further directed to a pharmaceutical composition comprising an inhibitor of the present invention and optionally a pharmaceutically acceptable carrier, excipient, dilutant or a combination thereof. In some embodiments, this pharmaceutical composition additionally comprises a chemotherapeutic such as platinum or gemcitabine, another disease specific active agent such as a fibrosis-modulating agent such as pirfenidone or nintendamib another oligonucleotide, an antibody and/or a small molecule which is for example effective in treatment of a disease selected from the group consisting of a fibrotic disease, an autoimmune disorder, an immune disorder, an acute inflammatory disease, a chronic inflammatory disease, a neurodegenerative disease and a tumor such as a malignant or benign tumor.

In some embodiments, the oligonucleotide of the present invention is in combination with another oligonucleotide, an antibody and/or a small molecule, either each of these compounds is separate or combined in a pharmaceutical composition, wherein the oligonucleotide, the antibody and/or the small molecule inhibits or stimulates a factor such as TGF-beta, PDGF, VEGF, EGF, FGF, wat, IGF-I, Notch, Hedgehog, Arginase 1 and 2, respectively, ICAM-1, VCAM-1, COL1A, ACTA2, ECAD, FN1 and/or Vim and a combination thereof.

Furthermore, the present invention relates to the use of the inhibitor or the pharmaceutical composition of the present invention in a method of preventing and/or treating a disorder, where an PRRX1 imbalance is involved. In some embodiments, the disorder is for example a fibrotic disease, an autoimmune disorder, an immune disorder, an acute inflammatory disease, a chronic inflammatory disease, a neurodegenerative disease and a tumor such as a malignant or benign tumor. In some embodiments, the inhibitor or the pharmaceutical composition of the present invention is for example administered locally or systemically.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### Description of figures

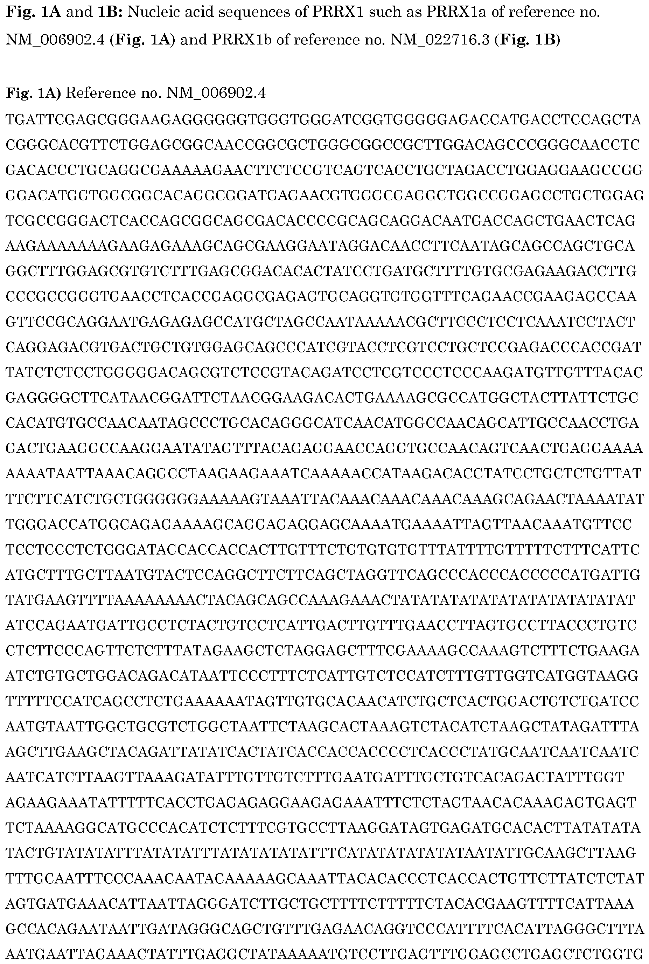

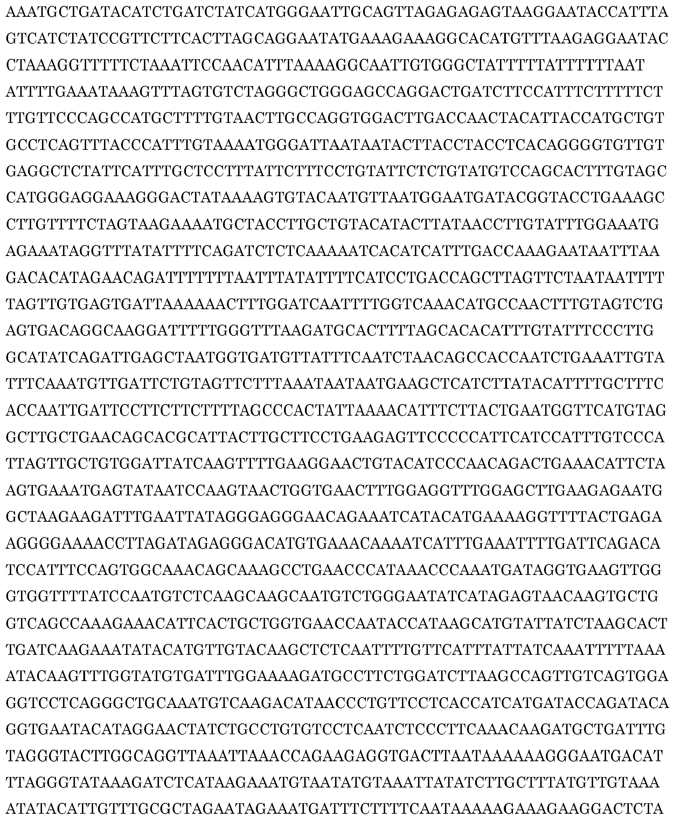

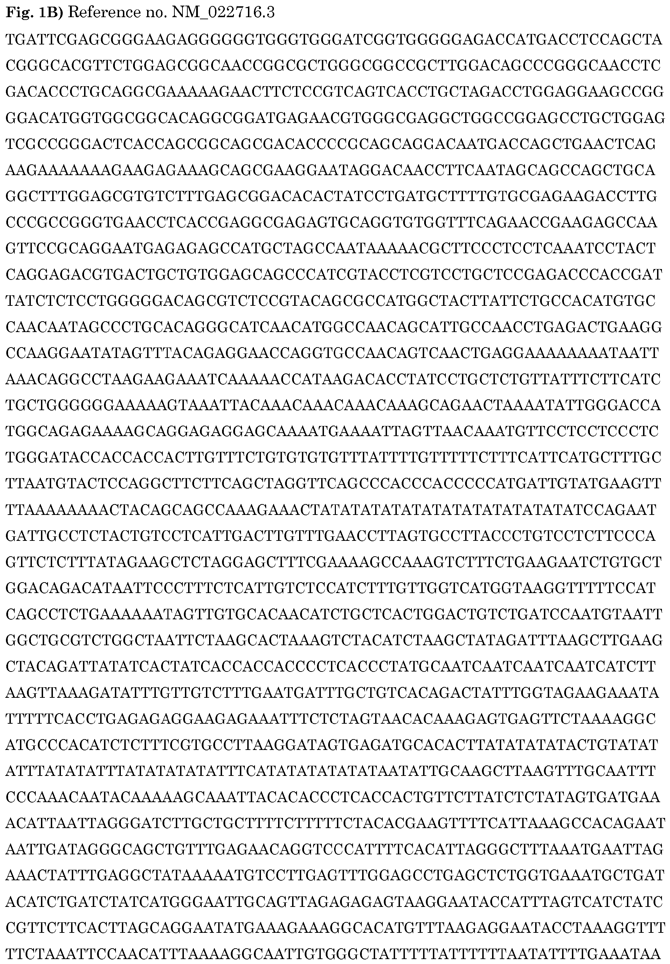

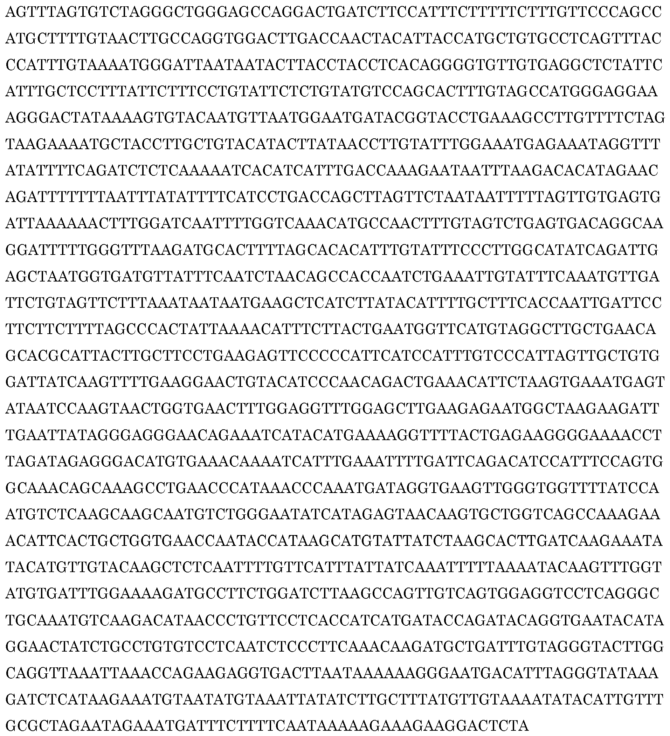

**Fig. 1A** **and** **1B** show the mRNA sequence of the two isoforms PRRX1a (pmx-1a; reference no. NM_006902.4) and PRRXlb (pmx-1b; reference no. NM_022716.3).

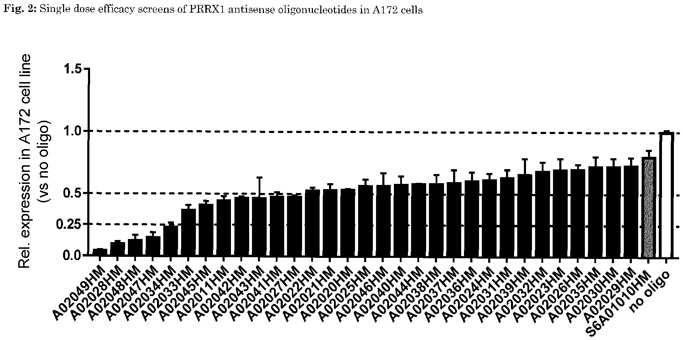

**Fig. 2** shows knock down efficacy of antisense oligonucleotides hybridizing with PRRX1 mRNA in the human glioma cell line A172. S6A01010HM is the negative control which is not complementary to any human or murine RNA. Cells were treated with oligonucleotides without any transfection reagent (gymnotic delivery) every second day for four days at a single concentration of 10 µM. After four days of treatment, cells were lysed and HPRT1 and PRRX1 mRNA expression was measured using the QuantiGene RNA Singleplex assay. PRRX1 expression values were normalized to HPRT1. Mean relative expression of PRRX1 mRNA (triplicates and standard deviation) compared to untreated cells (no oligo, set as 1) is depicted in **Fig. 2** for A172 cell line.

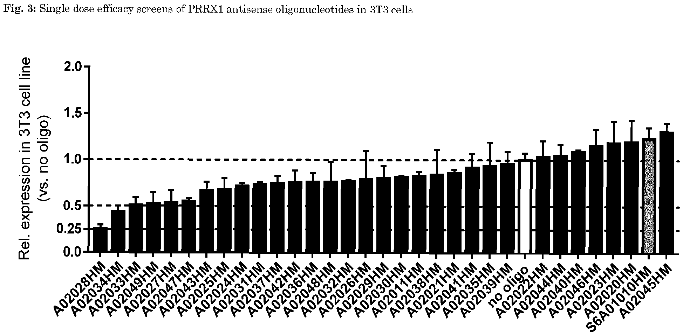

**Fig. 3** depicts knock down efficacy of antisense oligonucleotides hybridizing with PRRX1 mRNA in the murine fibroblast cell line 3T3. S6A01010HM is the negative control which is not complementary to any human or murine RNA. Cells were treated with oligonucleotides without any transfection reagent (gymnotic delivery) for three days at a single concentration of 10 µM. After three days of treatment, cells were lysed and HPRT1 and PRRX1 mRNA expression was measured using the QuantiGene RNA Singleplex assay. PRRX1 expression values were normalized to HPRT1. Mean relative expression of PRRX1 mRNA (triplicates and standard deviation) compared to untreated cells (no oligo, set as 1) is depicted in **Fig. 3** for 3T3 cell line.

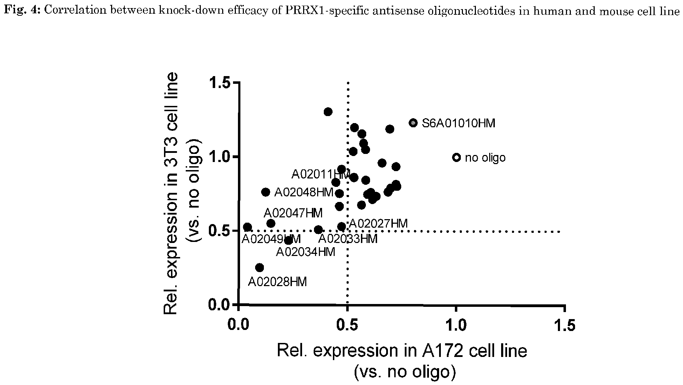

**Fig. 4** depicts correlation between knock-down efficacy of PRRX1-specific antisense oligonucleotides in human and mouse cell line. Mean relative expression of mouse (y axis) and human (x axis) PRRX1 mRNA (triplicates) compared to untreated cells (no oligo, set as 1) is depicted. Dotted line at 0.5 indicates 50 % knock-down efficacy.

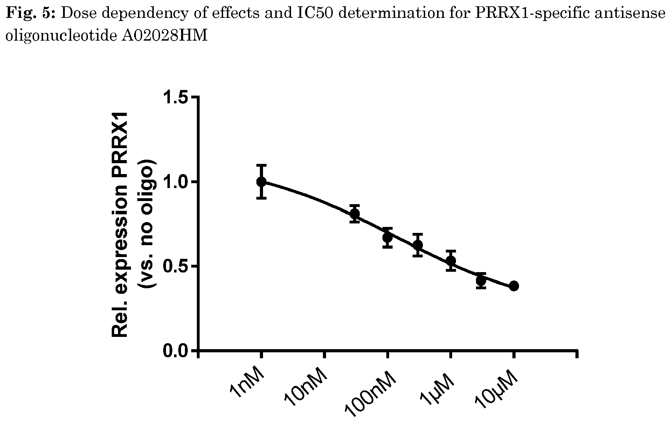

**Fig. 5** shows concentration-dependent PRRX1 mRNA knockdown by the PRRX1 antisense oligonucleotide A02028HM (SEQ ID NO.4) in 3T3 cells and IC₅₀ determination. 3T3 cells were treated for 3 days with the indicated concentration of the antisense oligonucleotide. PRRX1 mRNA expression values were normalized to expression of the housekeeping gene HPRT1. Normalized mPRRX1 mRNA expression relative to vehicle treated cells (set as 1) is depicted. IC₅₀ for A02028HM is 1.48 µM.

### Detailed description

The present invention provides for the first time human and murine antisense oligonucleotides which hybridize with mRNA sequences of PRRX1 such as PRRX1a and/or PRRXlb and inhibit the expression and activity, respectively, of PRRX1. Thus, the oligonucleotides of the present invention represent an interesting and highly efficient tool for use in a method of preventing and/or treating disorders, where the PRRX1 expression and activity, respectively, is increased. The oligonucleotide of the present invention hybridizes for example with a nucleic acid sequence of PRRX1 such as PRRX1a and/or PRRXlb of SEQ ID NO.1 and/or SEQ ID NO. 2, wherein the oligonucleotide inhibits at least 50 % of the PRRX1 expression. PRRX1a and PRRXlb represent isoforms of PRRX1, wherein PRRX1a is based on PRRX1b. The oligonucleotides hybridize with PRRX1 mRNA of human, mouse and rat, respectively.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

An oligonucleotide of the present invention is for example an antisense oligonucleotide (ASO) consisting of or comprising 10 to 25 nucleotides, 10 to 15 nucleotides, 15 to 20 nucleotides, 12 to 18 nucleotides, or 14 to 17 nucleotides. The oligonucleotides for example consist of or comprise 10, 11, 12, 13, 14, 15, 16, 17, 18, 19, 20, 21, 22, 23, 24 or 25 nucleotides. The oligonucleotides of the present invention comprise at least one nucleotide which is modified. The modified nucleotide is for example a bridged nucleotide such as a locked nucleic acid (LNA, e.g., 2',4'-LNA), cET, ENA, a 2'Fluoro modified nucleotide, a 2'O-Methyl modified nucleotide, a 2'O-Methoxy modified nucleotide, a FANA or a combination thereof. In some embodiments, the oligonucleotide of the present invention comprises nucleotides having the same or different modifications. In some embodiments the oligonucleotide of the present invention comprises a modified phosphate backbone, wherein one or more phosphate(s), e.g., all, is/are for example a phosphorothioate, a methylphosphonate or combinations thereof in the backbone. A nucleotide forms the building block of an oligonucleotide, and is for example composed of a nucleobase (nitrogenous base, e.g., purine or pyrimidine), a five-carbon sugar (e.g., ribose, 2-deoxyribose, arabinose, xylose, lyxose, allose, altrose, glucose, mannose, gulose, idose, galactose, talose or stabilized modifications of those sugars), and one or more phosphate groups. Examples of modified phosphate groups are phosphorothioate or methylphosphonate. Each compound of the nucleotide is modifiable, and is naturally or non-naturally occurring. Examples of the latter are: locked nucleic acid (LNA), 2', 4' constrained ethyl nucleic acids (c-ET), 2'-0,4'-C-ethylene-bridged nucleic acid (ENA), polyalkylene oxide- (such as triethylene glycol (TEG)), 2'-fluoro-, 2'-deoxy-2'-fluoro-beta-D-arabinonucleic acid (FANA), 2'-O-methoxy- and 2'-O-methyl-modified nucleotides.

An "LNA" is a modified RNA nucleotide, wherein the ribose moiety is modified with an extra bridge connecting the 2' oxygen and 4' carbon (2'- 4 'ribonucleoside). The bridge locks the ribose in the 3'-endo (North) conformation, which is often found in the A-form duplexes. LNA nucleosides and nucleotides, respectively, comprise for example the forms of thio-LNA, oxy-LNA, or amino-LNA, in alpha-D- or beta-L-configuration, and can be mixed or combined, respectively, with DNA or RNA residues in the oligonucleotide. A "bridged nucleic acid" is modified RNA nucleotide, sometimes also referred to as constrained or inaccessible RNA molecule, which may contain a five-membered, six-membered or even a seven-membered bridged structure with a "fixed" C3'-endo sugar puckering. The bridge is synthetically incorporated at the 2', 4'-position of the ribose to afford a 2', 4'-BNA monomer. Specific examples are "ENA" nucleotides, wherein the bridge is an ethylene bridge.

Oligonucleotides comprising modified nucleotides in specific regions having unmodified nucleotides in-between are entitled as "gapmers". The term "gapmer" for example refers to a chimeric antisense oligonucleotide that contains a central block of deoxynucleotide monomers sufficiently long to induce RNase H cleavage. The central block of a gapmer is for example flanked by blocks of 2'-O modified ribonucleotides or other artificially modified ribonucleotide monomers such as bridged nucleic acids (BNAs) that for example protect the internal block from nuclease degradation. In many earlier studies modified DNA analogs were investigated for their stability in biological fluids.

The oligonucleotide of the present invention comprises the one or more modified nucleotide at the 3'- and/or 5'- end of the oligonucleotide and/or at any position within the oligonucleotide, wherein modified nucleotides follow in a row of 1, 2, 3, 4, 5, or 6 modified nucleotides, or a modified nucleotide is combined with one or more unmodified nucleotides. For example the oligonucleotide of the present invention comprises at least one modified nucleotide, particularly at least one LNA, c-ET and/or ENA, at the 5'- and/or 3'-end of the oligonucleotide; or the oligonucleotide comprises 1, 2, 3, or 4 LNAs or c-ETs or ENAs within the stretch of up to 5 nucleotides at the 5'-end, and 1, 2, 3, or 4 LNAs or c-ETs or ENAs within the stretch of up to 5 nucleotides at the 3 '-end; or the oligonucleotide comprises 1, 2, 3, or 4 LNAs, c-ETs, or ENAs within the stretch of 5 nucleotides at the 5'-end or 3'-end, and a polyalkylene oxide such as TEG within the stretch of 5 nucleotides at the 3'- or 5'-end.

The following Table 1 presents embodiments of oligonucleotides comprising modified nucleotides for example LNA which are indicated by (+) and phosphorothioate (PTO) indicated by (*). The oligonucleotides consisting of or comprising the sequences of Table 1 may comprise any other modified nucleotide and/or any other combination of modified and unmodified nucleotides. Oligonucleotides of Table 1 hybridize with mRNA of human and/or murine PRRX1:

**Table 1: List of antisense oligonucleotides hybridizing with human and murine PRRX1, respectively, for example of SEQ ID No. 1 and/or 2; S6A01010HM is a negative control oligonucleotide that is not complementary to any human or murine RNA.**

| Seq ID | Name | Antisense Sequence 5'-3' | Antisense Sequence 5'-3' with PTO (*) and LNA (+) |
|---|---|---|---|
| 3 | A02049HM | TAGGTGTCTTATGGTT | +T*+A*+G*G*T*G*T*C*T*T*A*T*G*+G*+T*+T |
| 4 | A02028HM | GTTCTTTTTCGCCTGC | +G*+T*+T*C*T*T*T*T*T*C*G*C*C*+T*+G*+C |
| 5 | A02048HM | GTTGACTGTTGGCACCT | +G*+T*T*G*A*C*T*G*T*T*G*G*C*A*+C*+C*+T |
| 6 | A02047HM | GTAGCCATGGCGCTGTA | +G*+T*+A*G*C*A*T*G*G*C*G*C*T*+G*+T*+A |
| 7 | A02034HM | AGGTGACTGACGGAG | +A*+G*+G*T*G*A*C*T*G*A*C*G*+G*+A*+G |
| 8 | A02033HM | GTGACTGACGGAGAA | +G*+T*+G*A*C*T*G*A*C*G*G*A*+G*+A*+A |
| 9 | A02045HM | ATGGCGCTGTACGGA | +A*+T*+G*G*C*G*C*T*G*T*A*C*+G*+G*+A |
| 10 | A02011HM | TCAGGTTGGCAATGCTG | +T*C*+A*+G*G*T*T*G*G*C*A*A*T*G*+C*+T*G |
| 11 | A02042HM | GGAGAGATAATCGGT | +*+G*+A*G*A*G*A*T*A*A*T*C*+G*+G*+T |
| 12 | A02043HM | AGGAGAGATAATCGGT | +*+G*+G*A*G*A*G*A*T*A*A*T*C*+G*+G*+T |
| 13 | A02041HM | GAGCAGGACGAGGTACG | +*+A*+G*C*A*G*G*A*C*G*A*G*G*T*+A*+C*G |
| 14 | A02027HM | GCCGGTTGCCGCTCCAG | +G*+C*C*G*G*T*T*G*C*C*G*C*T*C*+C*+A*+G |
| 15 | A02022HM | CCGTAGCTGGAGGTCA | +C*+C*G*T*A*G*C*T*G*G*A*G*G*T*+C*+A |
| 15 | A02021HM | CCGTAGCTGGAGGTCA | +C*+C*+G*T*A*G*C*T*G*G*A*G*G*T*+C*+A |
| 15 | A02020HM | CCGTAGCTGGAGGTCA | +C*+C*+G*T*A*G*C*T*G*G*A*G*G*+T*+C*+A |
| 16 | A02025HM | TTGCCGCTCCAGAACG | +T*+T*+G*C*C*G*C*T*C*C*A*G*A*+A*+C*+G |
| 17 | A02046HM | CATGGCGCTGTACGGA | +C*+A*+T*G*G*C*G*C*T*G*T*A*C*+G*+G*+A |
| 18 | A02040HM | GCAGGACGAGGTACGAT | +G*+C*+A*G*G*A*C*G*A*G*G*T*A*C*+G*+A*+T |
| 19 | A02044HM | TGGCGCTGTACGGAGA | +T*+G*+G*C*G*C*T*G*T*A*C*G*G*+A*+G*+A |
| 20 | A02038HM | ACGTCTCCTGAGTAG | +A*+C*+G*T*C*T*C*C*T*G*A*G*+T*+A*+G |
| 21 | A02037HM | GCGGAACTTGGCTCT | +G*+C*+G*G*A*A*C*T*T*G*C*+T*+C*+T |
| 22 | A02036HM | CTAGCAGGTGACTGAC | +C*+T*+A*G*C*A*G*G*T*G*A*C*T*+G*+A*+C |
| 23 | A02024HM | TGCCGCTCCAGAACGTG | +T*+G*+C*C*G*C*T*C*C*A*G*A*A*C*+G*+T*+G |
| 24 | A02031HM | TGACTGACGGAGAAGTT | +T*+G*+A*C*T*G*A*C*G*G*A*G*A*A*+G*+T*+T |
| 25 | A02039HM | CACAGCAGTCACGTCTC | +C*+A*+C*A*G*C*A*G*T*C*A*C*G*T*+C*+T*+C |
| 26 | A02032HM | TGACTGACGGAGAAGT | +T*+G*+A*C*T*G*A*C*G*G*A*G*A*+A*+G*+T |
| 27 | A02023HM | CCGTAGCTGGAGGTC | +C*C*G*T*A*G*C*T*G*G*A*G*G*+T*+C |
| 28 | A02026HM | TTGCCGCTCCAGAAC | +T*+T*+G*C*C*G*C*T*C*C*A*G*+A*+A*+C |
| 29 | A02035HM | TAGCAGGTGACTGACG | +T*+A*+G*C*A*G*G*T*G*A*C*T*G*+A*+C*+G |
| 30 | A02030HM | CTGACGGAGAAGTTC | +C*+T*+G*A*C*G*G*A*G*A*A*G*+T*+T*+C |
| 31 | A02029HM | ACGGAGAAGTTCTTTT | +A*+C*+G*G*A*G*A*A*G*T*T*C*T*+T*+T*+T |
| 32 | S6A01010HM | TCTATCGTGATGTTTCT | +T*+C*+T*A*T*C*G*T*G*A*T*G*T*T*+T*+C*+T |

The following Table 2 shows knock down effectiveness of the antisense oligonucleotides of Table 1 on PRRX1 expression for example in human A172 and murine 3T3 cells, respectively:

**Table 2: Results of PRRX1 antisense oligonucleotides of Table 1 in inhibiting PRXX1 expression in human and murine cells; negative control is S6A01010HM antisense oligonucleotide.**

| Seq ID | Name | Position on mRNA | % Residual PRRX1 mRNA in human A172 cells | % Residual PRRX1 mRNA in murine 3T3 cells |
|---|---|---|---|---|
| 3 | A02049HM | 825 | 4,05 | 52,59 |
| 4 | A02028HM | 131 | 9,64 | 25,37 |
| 5 | A02048HM | 766 | 12,32 | 76,26 |
| 6 | A02047HM | 642 | 14,77 | 55,18 |
| 7 | A02034HM | 149 | 22,85 | 43,47 |
| 8 | A02033HM | 147 | 36,56 | 50,86 |
| 9 | A02045HM | 638 | 40,89 | 130,59 |
| 10 | A02011HM | 785 | 44,51 | 83,07 |
| 11 | A02042HM | 609 | 46,22 | 75,47 |
| 12 | A02043HM | 609 | 46,27 | 66,84 |
| 13 | A02041HM | 584 | 47,18 | 91,81 |
| 14 | A02027HM | 72 | 47,28 | 53,20 |
| 15 | A02022HM | 49 | 52,46 | 103,93 |
| 15 | A02021HM | 49 | 52,86 | 86,33 |
| 15 | A02020HM | 49 | 53,07 | 120,00 |
| 16 | A02025HM | 68 | 56,55 | 67,71 |
| 17 | A02046HM | 638 | 56,55 | 115,73 |
| 18 | A02040HM | 582 | 57,26 | 109,28 |
| 19 | A02044HM | 636 | 58,19 | 105,25 |
| 20 | A02038HM | 548 | 58,32 | 84,47 |
| 21 | A02037HM | 483 | 59,07 | 74,84 |
| 22 | A02036HM | 153 | 60,59 | 76,15 |
| 23 | A02024HM | 66 | 61,51 | 71,41 |
| 24 | A02031HM | 144 | 63,12 | 73,47 |
| 25 | A02039HM | 556 | 65,83 | 96,14 |
| 26 | A02032HM | 145 | 68,46 | 76,62 |
| 27 | A02023HM | 50 | 69,37 | 119,00 |
| 38 | A02026HM | 69 | 69,81 | 79,16 |
| 29 | A02035HM | 152 | 72,16 | 93,47 |
| 30 | A02030HM | 143 | 72,21 | 81,73 |
| 31 | A02029HM | 139 | 72,56 | 80,27 |
| 32 | S6A01010HM | - | - | - |

The oligonucleotides of the present invention hybridize for example with mRNA of human and/or murine PRRX1 of SEQ ID No. 1 and 2, respectively. Such oligonucleotides are called PRRX1 antisense oligonucleotides.

In some embodiments, the oligonucleotide of the present invention inhibits for example at least about 50%, 55%, 60%, 65%, 70%, 75%, 80%, 85%, 90%, 92%, 94%, 95%, 96%, 97%, 98%, 99% or 100% of PRRX1 such as the, e.g., human, rat and/or murine, PRRX1 expression. Thus, the oligonucleotides of the present invention are for example fibrosis-modulating, i.e., inhibiting or stimulating oligonucleotides which inhibit and revert fibrosis, respectively, for example in a cell, tissue, organ, or a subject. The oligonucleotide of the present invention inhibits the expression of PRRX1 at a nanomolar or micromolar concentration for example in a concentration of 0.1, 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900 or 950 nM, or 1,1.5, 2.0, 2.5, 3.0, 3.5, 10 or 100 µM.

In some embodiments, the oligonucleotide of the present invention is used in a concentration of 10, 20,,30, 40, 50, 60, 70, 80, 90, 100, 250, 300, 500, or 1000 nM, or 1, 1.5, 2.0, 2.2, 3, 5, 6.6 or 10 µM.

In some embodiments the present invention refers to a pharmaceutical composition comprising an oligonucleotide of the present invention and a pharmaceutically acceptable carrier, excipient and/or dilutant. In some embodiments, the pharmaceutical composition further comprises a chemotherapeutic, another disease specific active agent such as pirfenidone or nitendanib, another oligonucleotide, an antibody and/or a small molecule which is for example effective in the prevention and/or treatment of an autoimmune disorder, an immune disorder, an acute inflammatory disease, a chronic inflammatory disease, a neurodegenerative disease and a tumor such as a malignant or benign tumor.

In some embodiments, the oligonucleotide or the pharmaceutical composition of the present invention is for use in a method of preventing and/or treating a disorder. The disorder is for example characterized by an PRRX1 imbalance, i.e., the PRRX1 level is increased in comparison to the level in a normal, healthy cell, tissue, organ or subject. The PRRX1 level is for example increased by an increased PRRX1 such as PRRX1 expression and activity, respectively. The PRRX1 level can be measured by any standard method such as immunohistochemistry, western blot, quantitative real time PCR or QuantiGene assay known to a person skilled in the art.

An oligonucleotide or a pharmaceutical composition of the present invention is administered locally or systemically for example orally, sublingually, nasally, inhalational, subcutaneously, intravenously, intraperitoneally, intramuscularly, intratumoral, intrathecal, transdermal, and/or rectal. Alternatively or in combination *ex vivo* treated immune cells are administered. The oligonucleotide is administered alone or in combination with another antisense oligonucleotide of the present invention and optionally in combination with another compound such as another oligonucleotide, an antibody, a small molecule and/or another disease specific agent such as pirfenidone or nitendanib. In some embodiments, the other oligonucleotide (i.e., not being part of the present invention), the antibody, and/or the small molecule are effective in preventing and/or treating a fibrotic disease, an autoimmune disorder, an immune disorder, diabetes, artheriosclerosis, a nephrological disorder and/or cancer. An oligonucleotide or a pharmaceutical composition of the present invention is used for example in a method of preventing and/or treating a fibrotic or chronic inflammatory disease. Examples of fibrotic diseases preventable and/or treatable by use of the oligonucleotide or pharmaceutical composition of the present invention are pulmonary fibrosis, idiopathic pulmonary fibrosis, scleroderma, cirrhosis, biliary atresia, cardiac fibrosis, atrial fibrosis, myocardial infarction, interstitial lung disease, cystic fibrosis, myelofibrosis, steatohepatitis, interstitial pneumonia, non-alcoholic fatty liver disease, glomerulosclerosis, nephrogenic systemic fibrosis, radiation- or chemotherapy- induced lung injury, ventricular hypertrophy, ventricular remodeling, arthrofibrosis, mediastinal fibrosis, retroperitoneal fibrosis, Crohn's disease, Dupuytren's contracture, glaucoma, keloids and scaring.

In some embodiments two or more oligonucleotides of the present invention are administered together, at the same time point for example in a pharmaceutical composition or separately, or on staggered intervals. In other embodiments, one or more oligonucleotides of the present invention are administered together with another compound such as another oligonucleotide (i.e., not being part of the present invention), an antibody, a small molecule and/or a chemotherapeutic, at the same time point for example in a pharmaceutical composition or separately, or on staggered intervals. In some embodiments of these combinations the oligonucleotide (i.e., not being part of the present invention), the antibody and/or small molecule inhibits (antagonist) any of the following TGF-beta, PDGF, VEGF, EGF, FGF, wat, IGF-I, Notch, Hedgehog, Arginase 1 and 2, respectively, ICAM-1, VCAM-1, COL1A, ACTA2, ECAD, FN1 and/or Vim and a combination thereof.

The inhibitor or the pharmaceutical composition of the invention inhibits or suppresses for example a profibrotic and/or proinflammatory factor, wherein the proinflammatory factor is for example selected from the group consisting of a TNF-alpha, IL1-alpha, IL1-beta, CCL2, IL33, IL25, IL4, IL13, IL9, TSLP and a combination thereof.

An antibody in combination with the oligonucleotide or the pharmaceutical composition of the present invention is for example an CXCR4 antibody (AD-114). A small molecule in combination with the oligonucleotide or the pharmaceutical composition of the present invention are for example pirfenidone or nitendanib.

A subject of the present invention is for example a mammalian, a bird or a fish.

### Examples

The following examples illustrate different embodiments of the present invention, but the invention is not limited to these examples. The following experiments are performed on cells endogenously expressing PRRX1, i.e., the cells do not represent an artificial system comprising transfected reporter constructs. Such artificial systems generally show a higher degree of inhibition and lower IC₅₀ values than endogenous systems which are closer to therapeutically relevant in *vivo* systems. Further, in the following experiments no transfecting agent is used, i.e., gymnotic delivery is performed. Transfecting agents are known to increase the activity of an oligonucleotide which influences the IC₅₀ value (see for example Zhang et al., Gene Therapy, 2011, 18, 326-333; Stanton et al., Nucleic Acid Therapeutics, Vol. 22, No. 5, 2012). As artificial systems using a transfecting agent are hardly or impossible to translate into therapeutic approaches and no transfection formulation has been approved so far for oligonucleotides, the following experiments are performed without any transfecting agent.

### Example 1: Single dose efficacy screens of PRRX1 ASOs in A172 cells

4500 A172 cells / well were seeded in 96-well flat bottom plates and treated with the respective PRRX1 antisense oligonucleotides of Table 1 (black bars), or negative control (grey bar) at a final concentration of 10 µM or vehicle treated (white bar). After three days, cells were lysed and human HPRT1 (hHPRT1) and human PRRX1 (hPRRX1) mRNA expression were measured using the QuantiGene RNA Singleplex assay. hPRRX1 expression values were normalized to hHPRT1. Mean relative expression of hPRRX1 mRNA (triplicates and standard deviation) compared to untreated cells (no oligo, set as 1) is depicted in **Fig. 2****.** Dotted lines at 0.5 and 0.25 indicate 50 and 75 % knock-down efficacy.

### Example 2: Single dose efficacy screens of PRRX1 ASOs in 3T3 cells

4000 3T3 cells / well were seeded in 96-well flat bottom plates and treated with the respective PRRX1 antisense oligonucleotides of Table 1 (black bars), or negative control (grey bar) at a final concentration of 10 µM or vehicle treated (white bar). After three days, cells were lysed and mouse HPRT1 (mHPRT1) and mouse PRRX1 (mPRRX1) mRNA expression were measured using the QuantiGene RNA Singleplex assay. mPRRX1 expression values were normalized to mHPRT1. Mean relative expression of mPRRX1 mRNA (triplicates and standard deviation) compared to untreated cells (no oligo, set as 1) is depicted in **Fig. 3****.** Dotted lines at 0.5 and 0.25 indicate 50 and 75 % knock-down efficacy.

### Example 3: Dose dependency of effects and IC50 determination for PRRX1-specific antisense oligonucleotide A02028HM

3T3 cells were treated with different concentrations of the respective PRRX1 antisense oligonucleotides of Table 1 for three days. After three days, cells were lysed and mHPRT1 and mPRRX1 mRNA expression was measured using the QuantiGene RNA Singleplex assay. mPRRX1 expression values were normalized to mHPRT1. Normalized mPRRX1 mRNA expression relative to vehicle treated cells (set as 1) is depicted in **Fig. 5****.** ICso for A02028HM is 1.48 µM.

## Claims

1. PRRX1 inhibitor consisting of an antisense oligonucleotide comprising 12 to 20 nucleotides, wherein at least one of the nucleotides is modified, and the oligonucleotide hybridizes with a nucleic acid sequence of PRRX1 such as PRRX1a and/or PRRXlb of SEQ ID NO.1 (human) and/or SEQ ID NO. 2 (mouse), wherein the oligonucleotide inhibits at least 50 % of the PRRX1 expression.

2. Inhibitor according to claim 1, wherein the modified nucleotide is selected from the group consisting of a bridged nucleic acid such as LNA, cET, ENA, 2'Fluoro modified nucleotide, 2'O-Methyl modified nucleotide, a 2'O-Methoxy modified nucleotide, a FANA and a combination thereof.

3. The inhibitor of claim 1 or 2 hybridizing with PRRX1 of SEQ ID.NO.1 or 2 comprising a sequence selected from the group consisting of SEQ ID NO.3, SEQ ID NO.43, SEQ ID NO.5, SEQ ID NO.6, SEQ ID NO.7, SEQ ID NO.8, SEQ ID NO.9, SEQ ID NO.10, SEQ ID NO.11, SEQ ID NO.12, SEQ ID NO.13, SEQ ID NO.14, SEQ ID NO.15, SEQ ID NO.16, SEQ ID NO.17, SEQ ID NO.18, SEQ ID NO.19, SEQ ID NO.20, SEQ ID NO.21, SEQ ID NO.22, SEQ ID NO.23, SEQ ID NO.24, SEQ ID NO.25, SEQ ID NO.26, SEQ ID NO.27, SEQ ID NO.28, SEQ ID NO.29, SEQ ID NO.30, SEQ ID NO.31 and a combination thereof.

4. The inhibitor of any one of claims 1 to 3, wherein the oligonucleotide is selected from the group consisting of +T*+A*+G*G*T*G*T*C*T*T*A*T*G*+G*+T*+T (SEQ ID NO.3), +G*+T*+T*C*T*T*T*T*T*C*G*C*C*+T*+G*+C (SEQ ID NO.4), +G*+T*T*G*A*C*T*G*T*T*G*G*C*A*+C*+C*+T (SEQ ID NO.5), +G*+T*+A*G*C*C*A*T*G*G*C*G*C*T*G*+T*+A (SEQ ID NO.6), +A*+G*+G*T*G*A*C*T*G*A*C*G*+G*+A*+G (SEQ ID NO.7), +G*+T*+G*A*C*T*G*A*C*G*G*A*+G*+A*+A (SEQ ID NO.8), +A*+T*+G*G*C*G*C*T*G*T*A*C*+G*+G*+A (SEQ ID NO.9), +T*C*+A*+G*G*T*T*G*G*C*A*A*T*G*+C*+T*G (SEQ ID NO.10), +G*+G*+A*G*A*G*A*T*A*A*T*C*+G*+G*+T (SEQ ID NO.11), +A*+G*+G*A*G*A*G*A*T*A*A*T*C*+G*+G*+T (SEQ ID NO.12), +G*+A*+G*C*A*G*G*A*C*G*A*G*G*T*+A*+C*+G (SEQ ID NO.13), +G*+C*C*G*G*T*T*G*C*C*G*C*T*C*+C*+A*+G (SEQ ID NO.14), +C*+C*G*T*A*G*C*T*G*G*A*G*G*T*+C*+A (SEQ ID NO.15), +C*+C*+G*T*A*G*C*T*G*G*A*G*G*T*+C*+A (SEQ ID NO.15), +C*+C*+G*T*A*G*C*T*G*G*A*G*G*T*+C*+A (SEQ ID NO.15), +T*+T*+G*C*C*G*C*T*C*C*A*G*A*+A*+C*+G (SEQ ID NO.16), +C*+A*+T*G*G*C*G*C*T*G*T*A*C*+G*+G*+A (SEQ ID NO.17), +G*+C*+A*G*G*A*C*G*A*G*G*T*A*C*+G*+A*+T (SEQ ID NO.18), +T*+G*+G*C*G*C*T*G*T*A*C*G*G*+A*+G*+A (SEQ ID NO.19), +A*+C*+G*T*C*T*C*C*T*G*A*G*+T*+A*+G (SEQ ID NO.20), +G*+C*+G*G*A*A*C*T*T*G*G*C*+T*+C*+T (SEQ ID NO.21), +C*+T*+A*G*C*A*G*G*T*G*A*C*T*+G*+A*+C (SEQ ID NO.22), +T*+G*+C*C*G*C*T*C*C*A*G*A*A*C*+G*+T*+G (SEQ ID NO.23), +T*+G*+A*C*T*G*A*C*G*G*A*G*A*A*+G*+T*+T (SEQ ID NO.24), +C*+A*+C*A*G*C*A*G*T*C*A*C*G*T*+C*+T*+C (SEQ ID NO.25), +T*+G*+A*C*T*G*A*C*G*G*A*G*A*+A*+G*+T (SEQ ID NO.26), +C*C*G*T*A*G*C*T*G*G*A*G*G*+T*+C (SEQ ID NO.27), +T*+T*+G*C*C*G*C*T*C*C*A*G*+A*+A*+C (SEQ ID NO.28), +T*+A*+G*C*A*G*G*T*G*A*C*T*G*+A*+C*+G (SEQ ID NO.29), +C*+T*+G*A*C*G*G*A*G*A*A*G*+T*+T*+C (SEQ ID NO:30), +A*+C*+G*G*A*G*A*A*G*T*T*C*T*+T*+T*+T (SEQ ID NO.31), and a combination thereof, wherein + indicates an LNA nucleotide and * indicates a phosphorothioate (PTO) linkage between the nucleotides.

5. The inhibitor of any one of claims 1 to 4, wherein the inhibitor inhibits the expression of PRRX1 at a nanomolar or micromolar concentration.

6. A pharmaceutical composition comprising an inhibitor of any one of claims 1 to 5 and a pharmaceutically acceptable carrier, excipient, dilutant or a combination thereof.

7. The pharmaceutical composition of claim 6, further comprising a fibrosis-modulating agent such as perfenidone and/or nitendanib, another active agent, another oligonucleotide, an antibody and/or a small molecule.

8. The pharmaceutical composition of claim 7, wherein the other oligonucleotide, the antibody and/or the small molecule inhibits or suppresses a profibrotic and/or proinflammatory factor.

9. The pharmaceutical composition of claim 8, wherein the factor is selected from the group consisting of TGF-beta, PDGF, VEGF, EGF, FGF, wat, IGF-I, Notch, Hedgehog, Arginase 1 and 2, respectively, ICAM-1, VCAM-1, COL1A, ACTA2, ECAD, FN1 and/or Vim.and a combination thereof.

10. The pharmaceutical composition of claim 8, wherein the proinflammatory factor is selected from the group consisting of a TNF-alpha, IL1-alpha, IL1-beta, CCL2, IL33, IL25, IL4, IL13, IL9, TSLP and a combination thereof.

11. The Inhibitor of any one of claims 1 to 5 or the pharmaceutical composition of any one of claims 6 to 9 for use in a method of preventing and/or treating a disorder, where an PRRX1 imbalance is involved.

12. The inhibitor or the pharmaceutical composition for use according to claim 11, wherein the disorder is a fibrotic disorder, an autoimmune disorder, an immune disorder, an acute inflammatory disease, a chronic inflammatory disease, a neurodegenerative disease and a tumor such as a malignant or benign tumor.

13. The inhibitor or the pharmaceutical composition for use according to claim 12, wherein the chronic inflammatory diseases is a fibrotic disease such as pulmonary fibrosis, idiopathic pulmonary fibrosis, scleroderma, cirrhosis, biliary atresia, cardiac fibrosis, atrial fibrosis, myocardial infarction, interstitial lung disease, cystic fibrosis, myelofibrosis, steatohepatitis, interstitial pneumonia, non-alcoholic fatty liver disease, glomerulosclerosis, nephrogenic systemic fibrosis, radiation- or chemotherapy- induced lung injury, ventricular hypertrophy, ventricular remodeling, athrofibrosis, mediastinal fibrosis, retroperitoneal fibrosis, Crohn's disease, Dupuytren's contracture, glaucoma, keloids and/or scaring.

14. The inhibitor or the pharmaceutical composition for use according to claim 12 or 13, wherein the inhibitor or the composition is suitable to be administered locally or systemically.
